# FASCICULE DE BREVET EUROPEEN

(11) **EP 1 615 656 B1**
(45) Date de publication et mention de la délivrance du brevet: **08.08.2007**
(21) Numéro de dépôt: 04742461.9
(22) Date de dépôt: 08.04.2004
(51) Int. Cl.: A61K 38/16, A61K 35/74

(54) **PRODUIT IMMUNOMODULATEUR OBTENU A PARTIR D'UNE CULTURE DE BIFIDOBACTERIUM ET COMPOSITIONS LE CONTENANT**
AUS EINER BIFIDOBAKTERIEN-KULTUR GEWONNENER IMMUNOMODULATOR UND DIESEN ENTHALTENDE ZUSAMMENSETZUNGEN
IMMUNOMODULATORY PRODUCT OBTAINED FROM A BIFIDOBACTERIUM CULTURE AND COMPOSITIONS CONTAINING THE SAME

(30) Priorité: 16.04.2003 FR 0304746; 26.03.2004 FR 0403158
(43) Date de publication de la demande: 18.01.2006
(73) Titulaire: COMPAGNIE GERVAIS-DANONE, F-92302 Levallois Perret (FR)
(72) Inventeur: PETAY, Valérie, F-59850 Nieppe (FR); LECROIX, Francis, F-59270 Godewaersvelde (FR); PERRIN, Emmanuel, F-59299 Boescheppe (FR); GONTIER, Charles, F-59800 Lille (FR); BLAREAU, Jean-Pierre, F-59114 Steenvoorde (FR); ROMOND, Marie-Bénédicte, F-59800 Lille (FR); SINGER, Elisabeth, F-59800 Lille (FR); ODOU, Marie-Françoise, F-59270 Godeswaersvelde (FR); DEMAILLY-MULLIE, Catherine, F-62450 Le Sars (FR)
(74) Mandataire: Goulard, Sophie
(86) Numéro de dépôt international: PCT/FR2004/000874
(87) Numéro de publication internationale: WO 2004/093898

(56) Documents cités:
- EP-A- 0 768 375
- WO-A-01/01785
- MULLIE C ET AL: "Partial characterization of bifidobacterium breve C50 cell-free whey compounds inducing modifications to the intestinal microflora" JOURNAL OF DAIRY SCIENCE, AMERICAN DAIRY SCIENCE ASSOCIATION. CHAMPAIGN, ILLINOIS, US, vol. 85, no. 6, juin 2002 (2002-06), pages 1383-1389, XP002269107 ISSN: 0022-0302
- LIEPKE CORNELIA ET AL: "Human milk provides peptides highly stimulating the growth of bifidobacteria" EUROPEAN JOURNAL OF BIOCHEMISTRY, vol. 269, no. 2, janvier 2002 (2002-01), pages 712-718, XP002288966 ISSN: 0014-2956
- YASUI HISAKO ET AL: "Protection against influenza virus infection of mice fed Bifidobacterium breve YIT4064" CLINICAL AND DIAGNOSTIC LABORATORY IMMUNOLOGY, vol. 6, no. 2, mars 1999 (1999-03), pages 186-192, XP002288967 ISSN: 1071-412X

### SEQUENCE LISTING

<110> COMPAGNIE GERVAIS-DANONE
   PETAY, Valérie
   LECROIX, Francis
   PERRIN, Emmanuel
   GONTIER, Charles
   BLAREAU, Jean-Pierre
   ROMOND, Marie-Bénédicte
   SINGER, Elisabeth
   ODOU, Marie-Françoise
   DEMAILLY-MULLIE, Catherine
<120> PRODUIT IMMUNOSTIMULANT OBTENU À PARTIR D'UNE CULTURE DE BIFIDOBACTERIUM ET COMPOSITIONS LE CONTENANT
<130> F191 EXT 189
<150> FR0304746
   <151> 2003-04-16
<150> FR0403158
   <151> 2004-03-26
<160> 12
<170> PatentIn version 3.1
<210> 1
   <211> 21
   <212> PRT
   <213> Bifidobacterium breve
<400> 1
   Arg Glu Leu Gly Ile Gly Thr Pro Ser Phe Leu His Asn Gly Gly Gln
   1               5                   10                  15
   Trp Tyr Ile Tyr Ala
               20
<210> 2
   <211> 13
   <212> PRT
   <213> Bifidobacterium breve
<220>
   <221> MISC_FEATURE
   <222> (10)..(10)
   <223> acide aminé quelconque
<400> 2
   Arg Val Leu Tyr Asn Pro Gly Gln Tyr Xaa Tyr Val Arg
   1               5                   10
<210> 3
   <211> 22
   <212> PRT
   <213> Bifidobacterium breve
<400> 3
   Glu Gln Ala Thr Ala Asn Gly Gln Val Ser Ser Gly Gln Gln Ser Thr
   1 5 10 15
   Gly Gly Ser Ala Ala Pro
   20
<210> 4
   <211> 21
   <212> DNA
   <213> Artificial sequence
<220>
   <223> PCR primer
<400> 4
   tcaatcatgg cctgtggtct g 21
<210> 5
   <211> 19
   <212> DNA
   <213> Artificial sequence
<220>
   <223> PCR primer
<400> 5
   aagctcttgg cgtccgagg 19
<210> 6
   <211> 24
   <212> DNA
   <213> Artificial sequence
<220>
   <223> probe
<400> 6
   tcgccagcaa cctgaatcaa cctg 24
<210> 7
   <211> 19
   <212> DNA
   <213> Artificial sequence
<220>
   <223> PCR primer
<400> 7
   aatggcagac agcttttcg 19
<210> 8
   <211> 18
   <212> DNA
   <213> Artificial sequence
<220>
   <223> PCR primer
<400> 8
   gatcatggcg tggttagc 18
<210> 9
   <211> 30
   <212> DNA
   <213> Artificial sequence
<220>
   <223> probe
<400> 9
   ttccacttta acccccgctt caatgagaac 30
<210> 10
   <211> 21
   <212> DNA
   <213> Artificial sequence
<220>
   <223> PCR primer
<400> 10
   tggcgctttt gactcaggat t 21
<210> 11
   <211> 21
   <212> DNA
   <213> Artificial sequence
<220>
   <223> PCR primer
<400> 11
   gggatgtttg ctccaaccaa c 21
<210> 12
   <211> 27
   <212> DNA
   <213> Artificial sequence
<220>
   <223> probe
<400> 12
   gccgtcgcct tcaccgttcc agttttt 27

## Description

La présente Invention est relative à un produit immunomodulateur obtenu à partir d'une culture de *Bifidobacterium,* à son utilisation, notamment à titre de médicament ou d'ingrédient alimentaire, ainsi qu'aux compositions pharmaceutiques ou alimentaires le contenant.

Le genre *Bifidobacterium* fait partie de la famille des *Actinomycetaceae* ; il regroupe des bacilles à Gram positif, anaérobies stricts, fermentant le glucose par la voie de la fructose 6-phosphate phosphocétolase. Leur pH optimal de croissance est compris entre 6 et 7, et leur température optimale de croissance est comprise entre 37 et 46°C.

Les bifidobactéries font partie de la flore intestinale humaine normale, et on leur reconnaît de nombreux effets bénéfiques pour la santé. Il est notamment connu que les nourrissons alimentés au sein, qui possèdent une flore intestinale dans laquelle les bifidobactéries prédominent, résistent mieux aux infections et présentent notamment un risque de diarrhée plus faible que les nourrissons nourris avec des préparations lactées industrielles classiques.

Le rôle des bifidobactéries dans cette résistance accrue aux infections n'a pas été complètement élucidé. Différentes études indiquent qu'elles possèdent un pouvoir immunomodulateur qui impliquerait des substances polysaccharidiques associées à la paroi bactérienne, ou sécrétées par les bactéries au cours de la fermentation anaérobie. Gomez et al., (FEMS Microbiol. Lett., 1988, 56, 47-52) décrivent l'effet immunomodulateur de fractions exocellulaires riches en polysaccharides produites par *Bifidobacterium adolescentis ;* la demande de brevet FR 2 652 590 décrit un exopolymère immunopotentiateur de nature polysaccharidique produit par une souche du continuum *Bifidobacterium infantis longum* ; Honoso et al. (Biosci. Biotech. Biochem., 1997, 61, 312-316 et Bioscience Microflora, 1998, 17, 97-104) décrivent des polysaccharides immunopotentiateurs produits par différentes espèces de *Bifidobacterium.* L'action immunomodulatrice des bifidobactéries se manifeste également par la régulation de la microflore intestinale, en particulier au détriment du développement d'espèces bactériennes pathogène. Romond et al. (Anaerobe, 1997, 3, 137-143 et J. Dairy Sci., 1998, 81, 1229-1235) décrivent ainsi des fractions riches en glycoprotéines, produites par *Bifidobacterium breve* en conditions de fermentation anaérobie, et induisent *in vivo* un effet régulateur de la microflore intestinale.

On trouve ainsi sur le marché de nombreux produits fermentés par des bifidobactéries, éventuellement associées à d'autres bactéries lactiques, et dont l'ingestion permet de bénéficier des effets immunomodulateurs des bifidobactéries et de leurs produits de fermentation.

Cependant, et dans le cas particulier de l'alimentation infantile, ceux-ci présentent l'inconvénient d'être trop acides et de présenter, notamment dans le cas des produits en poudre, un aspect non-homogène après reconstitution, du fait de la coagulation des protéines du lait par l'acidité générée lors de la fermentation. Ils sont donc parfois mal acceptés par l'enfant et par la mère.

Afin de remédier à ces inconvénients, il a déjà été proposé dans la demande internationale WO 01/01785, un procédé de préparation d'un produit lacté immunostimulant par bioconversion, sans fermentation et donc sans acidification du produit final, d'un substrat laitier à l'aide de bifidobactéries, et en particulier de la souche *Bifidobacterium breve,* déposée selon le Traité de Budapest, le 31 mai 1999, sous le numéro I-2219 auprès de la CNCM (Collection Nationale de Cultures de Microorganismes) tenue par l'Institut Pasteur, 25 rue du Docteur Roux, à Paris.

Cependant, le procédé de préparation décrit dans cette demande internationale ne permet pas de préparer des produits alimentaires autres que des produits lactés et nécessite des conditions de mise en oeuvre contraignantes d'un point de vue industriel, notamment le maintien de conditions de culture aérobies, le maintien du milieu de culture à une pression osmotique correspondant à 0,93 à 0,97 d'activité de l'eau (AW), et/ou le maintien du milieu de culture à une température comprise entre 40 et 48°C.

Par ailleurs, les bactéries du genre *Bacteroides fragilis* représentent environ de 30 à 50 % de la flore présente dans les matières fécales chez l'homme. Leur localisation est majoritairement au niveau du colon (10¹¹ bactéries par gramme de selles). Cependant, en cas de prolifération anormale, les bactéries du genre *Bacteroides fragilis* sont responsables de 80 % des infections bactériennes anaérobies et sont de plus en plus fréquentes du fait de leur résistance croissante aux traitements antibiotiques. Leur prolifération anormale dans l'organisme peut entraîner :
- la formation d'abcès (abdominal, du cerveau, du foie, pelvien, poumon, rate),
- des septicémies,
- des diarrhées, principalement chez les jeunes enfants,
- des endocardites,
- des péritonites,
- des pneumonies, notamment nécrosantes.

Un taux de mortalité de 60 % en cas d'absence de traitement des infections à *Bacteroides fragilis* a été reporté.

Il peut donc être intéressant de pouvoir disposer d'un produit permettant de contrôler la prolifération de *Bacteroides fragilis.*

C'est donc afin de remédier à l'ensemble des inconvénients que présentent les produits décrits dans l'art antérieur et de pourvoir à un produit immunomodulateur pouvant être incorporé dans tout type de produit alimentaire ou être utilisé pour la préparation de compositions pharmaceutiques immunomodulatrices, que les Inventeurs ont mis au point ce qui fait l'objet de l'Invention.

Les Inventeurs se sont également donné pour but de pourvoir à une composition alimentaire ou pharmaceutique ayant un effet régulateur de la microflore intestinale, en particulier au détriment du développement d'espèces bactériennes pathogènes, notamment *Bacteroides fragilis.*

La présente Invention a donc pour premier objet un produit immunomodulateur caractérisé par le fait qu'il est obtenu selon un procédé de préparation comprenant les étapes suivantes :
- l'ensemencement et l'incubation, en conditions aérobies ou anaérobies, préférentiellement anaérobies, et à une température comprise entre 30 et 40°C environ, de *Bifidobacterium* comprenant au moins la souche *Bifidobacterium breve* I-2219 dans un substrat aqueux présentant un pH compris entre 6 et 8 environ, et comprenant au moins les ingrédients suivants :
   i) du perméat de lactosérum,
   ii) un hydrolysat de protéines de lactosérum,
   iii) du lactose,
- l'élimination des *Bifidobacterium* du substrat aqueux ;
- l'ultrafiltration du substrat aqueux sur des membranes de filtration ayant un seuil de coupure compris entre 100 et 300 kDa pour obtenir un rétentat concentré ;
- la déshydratation du rétentat concentré, de préférence par lyophilisation ;
- la mise en solution du rétentat déshydraté dans un tampon ;
- la chromatographie d'exclusion sur gel sur colonne présentant un seuil d'exclusion de 600 kDa de la solution du rétentat ;
- la récupération de la fraction exclue à l'issue de la chromatographie qui constitue le produit immunomodulateur.

La fraction exclue obtenue en mettant en oeuvre le procédé conforme à l'Invention présente des propriétés immunomodulatrices ; elle permet en particulier de stimuler la prolifération des *Bifidobacterium* et de diminuer la population en *Bacteroïdes fragilis* au sein de la flore intestinale.

Selon l'Invention, le perméat de lactosérum entrant dans la composition du substrat aqueux peut se présenter sous la forme d'une poudre, obtenue par ultrafiltration de lactosérum, après séchage (par spray ou par toute autre technique de séchage), de la fraction liquide, déminéralisée ou non, qui franchit la membrane lors de l'ultrafiltration du lactosérum.

Les hydrolysats de protéines de lactosérum utilisables dans le cadre de la présente Invention, peuvent être obtenus par les méthodes habituellement utilisées pour la préparation des hydrolysats de protéines, notamment par hydrolyse enzymatique des protéines de lactosérum à l'aide de protéases telles que la trypsine, la chymotrypsine, etc... De nombreux concentrés ou isolats de protéines sériques de même que des hydrolysats de protéines de lactosérum, convenant pour la mise en oeuvre de l'Invention sont connus en eux-mêmes et disponibles dans le commerce.

Avant son utilisation, le substrat aqueux est de préférence filtré sur membranes, telles que par exemple sur des membranes en polyéthersulfone, ayant un seuil de coupure compris entre 100 et 300 kDa, puis le perméat autoclavé à une température d'environ 120°C pendant environ 30 minutes de façon à éviter toute contamination bactérienne indésirable du milieu culture.

Avant emploi, le pH du substrat aqueux peut être ajusté à la valeur désirée à l'aide de tout agent alcalinisant classiquement utilisé par l'homme du métier tel que par exemple la soude ou la potasse.

Les *Bifidobacterium* sont, de préférence, ensemencés dans le substrat aqueux à raison de 1.10⁴ à 4.10⁹ unités formant colonies (UFC) par ml de substrat. Cet ensemencement peut s'effectuer par exemple par addition au substrat aqueux, dans des proportions adaptées, d'un concentré congelé de *Bifidobacterium,* ou d'une pré culture sur milieu permettant la croissance de bifidobactéries.

Selon une forme de réalisation préférée de l'Invention, le pH du substrat aqueux est maintenu à une valeur comprise entre 6 et 8 environ pendant toute la période d'incubation, et encore plus préférentiellement entre 6,5 et 7,5. Le maintien du pH est de préférence réalisé par une neutralisation en continu du substrat aqueux à l'aide d'un agent alcalinisant tel que décrit précédemment ou par une solution d'ammoniaque diluée (de préférence au demi).

Selon une forme de réalisation préférée de l'Invention, la température du substrat est maintenue à une valeur comprise entre 37 et 40°C environ pendant toute la durée de l'incubation, celle-ci étant généralement comprise entre 10 et 20 heures.

Selon une forme de réalisation préférée du procédé conforme à l'Invention, les ingrédients du substrat aqueux sont présents dans les quantités suivantes :
i) perméat de lactosérum : de 3 à 80 g environ et encore plus préférentiellement de 40 à 60 g environ,
ii) hydrolysat de protéines de lactosérum : de 2 à 80 g environ et encore plus préférentiellement de 5 à 15 g environ,
iii) lactose : de 5 à 50 g environ et encore plus préférentiellement de 10 à 30 g environ,
   ces quantités étant données par litre dudit substrat aqueux.

Selon une forme de réalisation particulière de l'Invention, le substrat aqueux peut comprendre en outre au moins un ingrédient additionnel choisi parmi les extraits de levure, les sels tampon et le chlorhydrate de cystéine.

Lorsque le substrat aqueux comprend un sel tampon, celui-ci est préférentiellement choisi parmi le dihydrogénophosphate de sodium et le dihydrogénophosphate de potassium et représente alors de préférence de 0,5 à 5 g environ et encore plus préférentiellement de 1,5 à 3 g environ par litre de substrat aqueux.

Lorsque le substrat aqueux comprend un extrait de levure, celui-ci représente alors de préférence de 0,5 à 5 g environ, et encore plus préférentiellement de 1,5 à 3 g environ par litre de substrat aqueux.

Lorsque le substrat aqueux comprend du chlorhydrate de cystéine, celui-ci représente alors de préférence de 100 à 500 mg environ et encore plus préférentiellement de 200 à 400 mg environ par litre de substrat aqueux.

A la fin de la période d'incubation, l'élimination des *Bifidobacterium* du milieu de culture peut être réalisée par exemple par microfiltration ou par centrifugation du substrat aqueux. Selon une forme de réalisation préférée de l'Invention, l'élimination des *Bifidobacterium* du milieu de culture est réalisée par centrifugation du substrat aqueux, par exemple à une vitesse de 3000 g pendant une durée d'environ 1 heure.

Selon une forme de réalisation préférée de l'Invention, le procédé comporte en outre, après l'étape d'élimination des *Bifidobacterium,* une étape supplémentaire de destruction des activités enzymatiques résiduelles contenues dans le substrat aqueux après incubation, par exemple par un traitement thermique de celui-ci à une température d'environ 75°C pendant environ 3 minutes.

L'étape d'ultrafiltration du substrat aqueux est de préférence réalisée sur des membranes en polyéthersulfone, à une température inférieure à 60°C environ.

A la fin de l'étape d'ultrafiltration, le rétentat concentré ainsi obtenu est de préférence lavé plusieurs fois, par exemple à l'eau permutée avant d'être finalement reconcentré avant d'être déshydraté par exemple par lyophilisation.

Le tampon utilisé pour la mise en solution du rétentat déshydraté est de préférence choisi parmi les tampons présentant un pH compris entre 6 et 8 tels que par exemple le tampon Tris ajusté au pH voulu par ajout d'acide chlorhydrique.

La nature des gels utilisables pour réaliser la chromatographie d'exclusion n'est pas critique à partir du moment que ceux-ci présentent un seuil d'exclusion de 600 kDa. A titre de gel on peut notamment utiliser les gels composés de dextrane et d'agarose réticulé tels que le produit vendu sous la dénomination commerciale Superdex® 200 par la société Amersham Biosciences.

Lorsque la chromatographie est terminée, la fraction exclue récupérée peut ensuite être dialysée contre de l'eau distillée puis éventuellement diluée de façon à revenir à la concentration initiale de la fraction exclue.

Enfin, la fraction exclue constituant le produit immunomodulateur peut être utilisée directement ou congelée ou lyophilisée pour conservation et utilisation ultérieure.

Cette fraction exclue est essentiellement constituée d'un complexe de polysaccharides et de protéines dans lequel la fraction glucidique représente de 5 à 30 % en poids environ, la fraction protéique représentant environ de 70 à 95 % en poids par rapport au poids total dudit complexe.

Selon l'Invention, la fraction glucidique de la fraction exclue présente la composition en monosaccharides suivante (exprimée en rapports molaires par rapport au rhamnose) : galactose : 5,5 à 8 ; mannose : 0,8 à 1,3 ; glucose: 2,5 à 5 ; N-acétyl galactosamine : 0,3 à 1; N-acétyl glucosamine : 0,07 à 0,3 ; acide neuraminique 0 à 0,15 et rhamnose : 1.

Selon l'Invention, la fraction protéique de la fraction exclue peut comprendre au moins un peptide, obtenu par hydrolyse trypsique, répondant à au moins l'une des séquences suivantes :
- RELGIGTPSFLHNGGQWYIYA (SEQ ID n°1)
- RVLYNPGQYXYVR (SEQ ID n°2)
- EQATANGQVSSGQQSTGGSAAP (SEQ ID n°3)

L'Invention a également pour objet le produit immunomodulateur obtenu selon le procédé décrit précédemment à titre de médicament, et en particulier à titre de médicament immunomodulateur.

Un autre objet de l'Invention est une composition pharmaceutique caractérisée par le fait qu'elle renferme, à titre de principe actif, au moins un produit immunomodulateur obtenu selon le procédé décrit précédemment, et au moins un support pharmaceutiquement acceptable.

Par pharmaceutiquement acceptable on entend tout support qui tout en conservant au produit immunomodulateur obtenu selon le procédé conforme à l'Invention ses propriétés, particulièrement ses propriétés immunomodulatrices, permet de véhiculer ledit produit.

La composition pharmaceutique selon l'Invention peut se présenter sous toute forme galénique souhaitée pour une administration par voie orale à l'homme ou à l'animal, comme par exemple sous forme liquide pour un sirop ou une solution, un spray, ou sous forme solide comme par exemple une poudre, un comprimé, une gélule, une capsule, un spray poudre, dans leurs formes diverses, libération immédiate ou programmée, une gomme, une pâte, des granulés, ou sous toute autre forme adaptée à l'administration par voie orale.

Le produit immunomodulateur obtenu selon le procédé conforme à l'Invention peut également être incorporé, à titre d'ingrédient, dans des compositions alimentaires.

Par conséquent, l'Invention a également pour objet une composition alimentaire caractérisée par le fait qu'elle renferme, à titre d'ingrédient, au moins un produit immunomodulateur obtenu selon le procédé conforme à l'Invention.

De telles compositions alimentaires peuvent être destinées à l'alimentation humaine ou animale et peuvent notamment se présenter sous forme d'une préparation lactée ou non, fermentée ou non, d'origine animale ou végétale, y compris notamment les formules infantiles ou pour adultes et seniors, et en particulier sous forme d'une préparation lactée infantile, de lait liquide ou en poudre, de produits frais, de céréales, de biscuits (fourrage), de petits pots, de desserts, etc, ou bien encore sous forme de produits alimentaires ou diététiques pour adultes, dont les produits hospitaliers, ou de compléments nutritionnels.

La présente Invention sera mieux comprise à l'aide du complément de description qui va suivre, qui se réfère à des exemples de préparation du produit immunomodulateur conforme à l'Invention, ainsi qu'aux figures annexées dans lesquelles :
- la figure 1 représente le chromatogramme obtenu après injection sur une colonne garnie d'un gel de Superdex® 200 d'un milieu de culture fermenté pendant 15 heures par la souche *Bifidobacterium breve* CNCM I-2219 (absorbance en millivolts en fonction du temps écoulé en minutes) ;
- la figure 2 compare les chromatogrammes obtenus après injection sur une colonne garnie d'un gel de Superdex® 200 d'un milieu de culture fermenté par la souche *Bifidobacterium breve* CNCM I-2219 ou par la souche *B. breve* CFPL (Collection de la Faculté de Pharmacie de Lille) C7 (absorbance en millivolts en fonction du temps écoulé en minutes) ;
- la figure 3 représente un agrandissement de la figure 2.

### EXEMPLE 1 : PREPARATION D'UN PRODUIT IMMUNOMODULATEUR OBTENU PAR CULTURE DE BIFIDOBACTERIUM

On prépare un milieu de culture contenant les ingrédients suivants :
- 50 g/l de perméat de lactosérum,
- 10 g/l d'hydrolysat de protéines de lactosérum,
- 20 g/l de lactose,
- 2 g/l d'extrait de levure,
- 2,5 g/l de dihydrogénophosphate de potassium,
- 0,3 g/l de chlorhydrate de cystéine.

Le milieu de culture est ultrafiltré sur cassettes Centramate® vendues par la société PALL, munies de membranes en polyéthersulfone ayant un seuil de coupure de 200 kDa et le perméat est autoclavé pendant 30 minutes à 120°C. Le pH du milieu de culture est alors ajusté à une valeur de 6,5 à l'aide d'une solution d'ammoniaque diluée au quart.

Le milieu de culture est ensuite ensemencé avec les bifidobactéries à raison de 6 °/oo (v/v) d'un concentré congelé de la souche de *Bifidobacterium breve* CNCM I-2219 contenant 5.10¹⁰ UFC de bifidobactéries par ml de concentré congelé. La population initiale en bactéries est de 3.10⁸ UFC de bifidobactéries par ml de milieu de culture. Les bifidobactéries sont cultivées en anaérobiose, à une température comprise entre 37 et 40°C. Pendant la culture, le pH du milieu de culture est régulé à 6,5 au moyen d'une solution d'ammoniaque diluée au quart. Le temps de culture est de 15 heures, et la population de *Bifidobacterium* en fin de culture est environ de 2.10⁷ UFC par ml de milieu de culture.

En fin de culture, les bactéries sont éliminées du milieu de culture fermenté par une centrifugation de 1 heure à 3000 g. Les activités enzymatiques résiduelles contenues dans le surnageant de centrifugation sont détruites par un traitement thermique de 3 minutes à 75°C.

Le surnageant est ultrafiltré sur cassettes Centramate® vendues par la société PALL, munies de membranes en polyéthersulfone ayant un seuil de coupure de 300 kDa à une température de 40°C environ. Il est ainsi concentré 3 fois, puis lavé 3 fois à l'eau permutée. Pendant le dernier lavage, on opère une concentration de 7 fois de la partie retenue par la membrane. On obtient ainsi un concentré appelé rétentat. Le rétentat est déshydraté par lyophilisation, puis repris dans un tampon Tris-NaCl à pH 8.

### 1) Etude de la composition du rétentat obtenu

La composition du rétentat est étudiée par chromatographie d'exclusion.

Pour ce faire, 25 µl de rétentat sont injectés à un débit de 0,6 ml par minute sur colonne de gel Superdex® 200 vendu par la société Amersham Biosciences et présentant un seuil d'exclusion de 600 kDa, couplée à un détecteur UV à barrette de diodes (200-300 nm). L'intégration du signal est réalisée à l'aide du logiciel KromaSystem® 2000 vendu par la société Kontron Instruments. Deux fractions sont ainsi séparées : une fraction exclue du gel est éluée après 12,5 minutes à partir de l'injection et une fraction filtrée est éluée de 16 à 32 minutes à partir de l'injection.

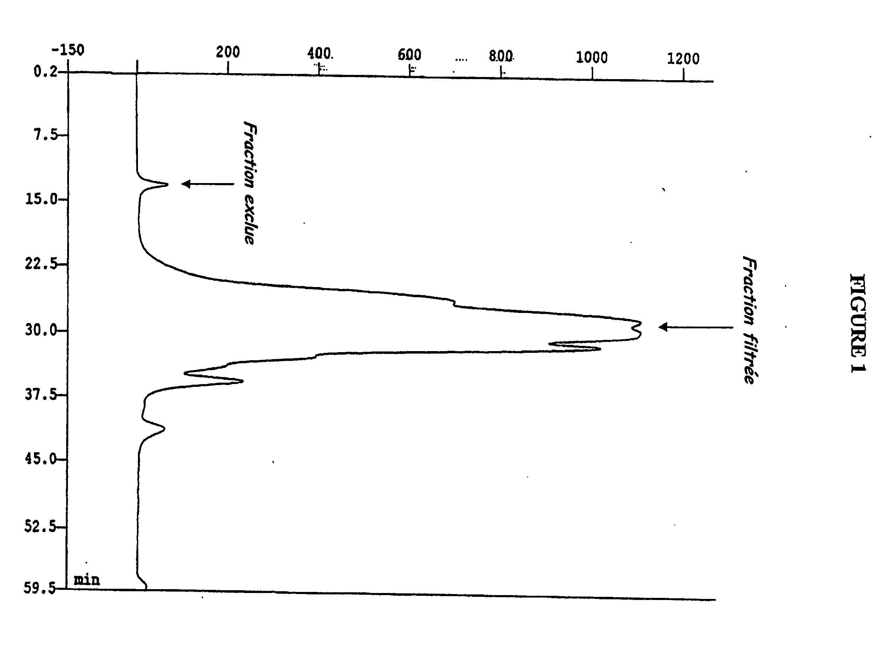

Les résultats obtenus sur le rétentat après 15 heures de fermentation sont reportés sur la figure 1 annexée qui représente l'absorbance (en millivolts) en fonction du temps écoulé (en minutes) depuis l'injection du rétentat sur la colonne. Ces résultats représentent un chromatogramme typique montrant la fraction exclue et la fraction filtrée du rétentat ainsi analysé.

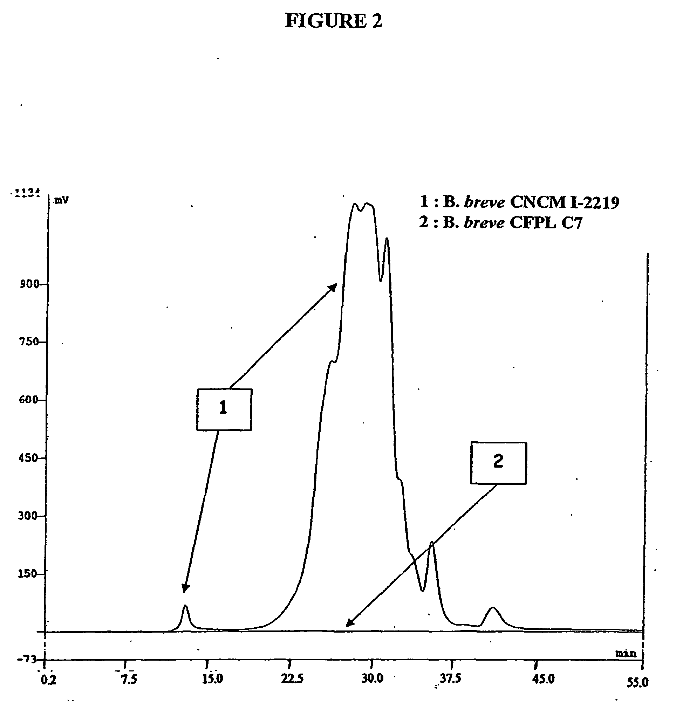

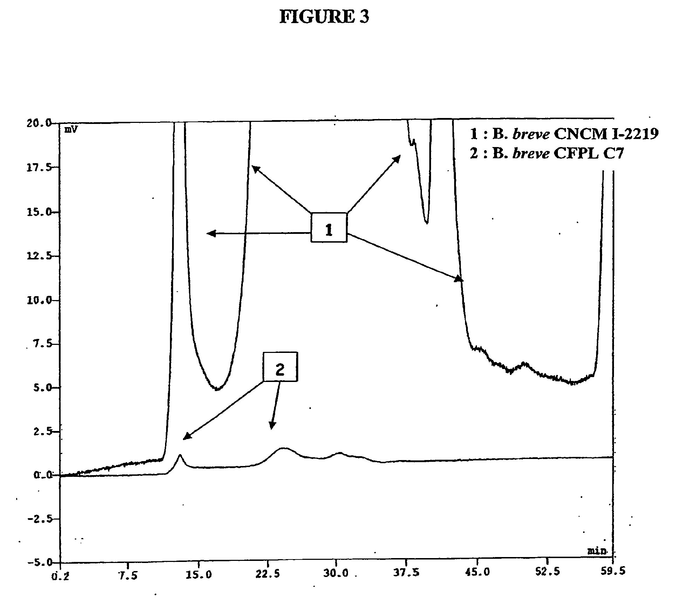

Les figures 2 et 3 représentent les chromatogrammes obtenus après analyse d'un rétentat issu d'une culture réalisée comme décrite précédemment et d'un rétentat issu d'une culture réalisée dans les mêmes conditions mais avec une souche différente de bifidobactéries (*B. breve* CFPL C7).

La figure 2 représente l'absorbance (en millivolts) en fonction du temps écoulé (en minutes) depuis l'injection sur la colonne des rétentats correspondants aux deux cultures réalisées respectivement avec la souche *B. breve* CNCM I-2219 et avec la souche *B. breve* CFPL C7. Ces résultats montrent que, contrairement au rétentat de la culture réalisée avec la souche de *B. breve* CNCM I-2219, le chromatogramme du rétentat de la culture réalisée avec la souche de *B. breve* CFPL C7 ne présente aucun pic correspondant à la fraction exclue et à la fraction filtrée du rétentat. En revanche, la figure 3 qui représente un agrandissement de la figure 2, montrent qu'il est nécessaire d'agrandir considérablement l'échelle d'absorbance de la figure 2 pour faire apparaître la fraction exclue et la fraction filtrée du rétentat correspondant à la culture réalisée avec la souche *B. breve* CFPL C7. Ces résultats démontrent ainsi que la souche *B. breve* CFPL C7 ne présente pas le potentiel de production de principes actifs que représente la souche *B. breve* CNCM I-2219.

### 2) Préparation de la fraction exclue du rétentat

Le rétentat concentré, préalablement repris dans un tampon Tris-NaCl pH 8, est soumis à une chromatographie préparative. La séparation est réalisée par chromatographie sur colonne de gel Superdex® 200 vendue par la société Amersham Biosciences de 50 mm de diamètre et 100 cm de hauteur, alimentée à un débit de 5 ml par minute et présentant un seuil d'exclusion de 600 kDa. Les fractions sont collectées par 10 ml et leur absorbance est mesurée à 280 nanomètres.

Deux fractions sont ainsi séparées :
- une fraction exclue du gel de poids moléculaire supérieur à 600 kDa (temps de rétention de 130 à 180 minutes +/- 10 %),
- une fraction filtrée de poids moléculaire compris entre 200 et 600 kDa (temps de rétention de 187 à 370 minutes +/- 10 %).

La fraction exclue ou partie active est dialysée contre de l'eau distillée puis diluée de façon à revenir à la concentration du rétentat. Cette fraction peut ensuite être conservée sous forme congelée ou lyophilisée. La fraction filtrée peut également être conservée de la même manière.

### EXEMPLE 2: ANALYSE DE LA COMPOSITION GLUCIDIQUE ET PROTEIOUE DE LA PARTIE ACTIVE (FRACTION EXCLUE) PREPAREE A PARTIR D'UNE CULTURE DE BIFIDOBACTERIUM

### 1) Analyse de la composition glucidique de la partie active

La poudre lyophilisée de fraction exclue telle que préparée ci-dessus à l'exemple 1 (10 mg) est reprise par de l'hydrazine anhydre pure (200-300 microlitres). Le mélange est incubé une nuit à 110°C, séché sous flux d'azote et repris dans 1 ml d'eau. La solution est ensuite fractionnée par perméation de gel sur colonne Fractogel TSK, vendue sous la référence HW40 par la société Merck.et l'élution est réalisée en eau. La présence de sucres dans les différentes fractions recueillies est recherchée par la méthode à l'orcinol et par chromatographie sur couche mince. Les résultats obtenus (non représentés) montrent que la majorité des sucres est dans la fraction non retenue sur le gel (>10 kDa) et migre peu après chromatographie sur couche mince. La partie glucidique de la fraction exclue constitue donc un polysaccharide de masse molaire supérieure à 10 kDa. La partie glucidique de la fraction exclue représente environ 15 à 20 % en masse de celle-ci.

La composition molaire de la fraction glucidique de la partie active est déterminée par chromatographie en phase gazeuse après méthanolyse à l'aide d'un mélange de 0,5 M d'acide hydrochlorique et de méthanol pendant 24 heures à 80°C.

Deux échantillons de la fraction exclue (Echantillons 1 et 2 CNCM I-2219 : respectivement E1 et E2), provenant de deux essais différents réalisés selon l'exemple 1, sont analysés.

A titre de comparaison, les mêmes analyses ont été réalisées sur un échantillon où la souche *Bifidobacterium breve* CNCM 1-2219 a été remplacée par la souche *Bifidobacterium breve* CFPL C7 (Echantillon C7) cultivée dans les mêmes conditions, ainsi que sur un échantillon où la souche *Bifidobacterium breve* CNCMI-2219 a été cultivée sur un milieu lactose non conforme à l'Invention (Echantillon milieu lactose : ML) de composition suivante :
- 60 g/l de lactose,
- 2 g/l d'extrait de levure,
- 0,3 g/l de chlorhydrate de cystéine.

Les étapes suivantes d'extraction et de purification étant comparables en tout point à celles décrites précédemment.

Les rapports molaires en monosaccharides donnés (exprimés par rapport au rhamnose) obtenus pour la fraction exclue de ces différents échantillons sont regroupés dans le Tableau I suivant :

**TABLEAU I**

| | **E1** | **E2** | **C7*** | **ML*** |
|---|---|---|---|---|
| **Galactose** | 6,50 | 7,60 | 2,00 | 3,80 |
| **Mannose** | 0,92 | 1,10 | 2,70 | 6,20 |
| **Glucose** | 3,20 | 4,30 | 3,50 | 2,70 |
| **N-acétyl galactosamine** | 0,80 | 0,47 | 1,00 | 0,10 |
| **N-acétyl glucosamine** | 0,17 | 0,10 | 0,38 | 0,10 |
| **Acide neuraminique** | 0,08 | - | 0,49 | - |
| **Rhamnose** | 1,00 | 1,00 | 0 | 1,00 |

| | | | | |
|---|---|---|---|---|
| *: échantillon comparatif ne faisant pas partie de l'Invention. | | | | |

On peut noter l'absence de rhamnose dans l'échantillon C7 où la souche *Bifidobacterium breve* CNCM I-2219 a été remplacée par la souche *Bifidobacterium breve* CFPL C7. On peut également constater que la répartition des sucres entre les échantillons E1 et E2 est différente de celle de l'échantillon ML correspondant à la souche *Bifidobacterium breve* CNCM I-2219 cultivée sur un milieu lactose non conforme à l'Invention. En particulier, les échantillons E1 et E2 renferment plus de galactose et de N-acétyl galactosamine que l'échantillon ML et moins de mannose que l'échantillon ML.

### 2) Analyse de la composition protéique de la partie active

La fraction protéique de la partie active représente environ 80 à 85 % en masse de celle-ci.

Le séquençage de la partie protéique de la partie active est réalisé après une étape de protéolyse à l'aide d'une solution de trypsine à 1 % dans un tampon Tris 0,1 M à pH 8.5, comme décrit dans l'article Rosenfeld J. et al., Analytical Biochemistry, 1992, 203, 173-179.

Les peptides sont purifiés par HPLC en phase inverse sur une colonne Ultrasphere® ODS (octadécylsilane) d'un diamètre de 2 mm et d'une longueur de 200 mm vendue par la société Beckmann. L'élution est effectuée par un gradient linéaire d'acétonitrile dans une solution d'acide trifluoroacétique à 0,1 %.

Les peptides isolés sont séquencés (selon la méthode décrite dans l'article de Rosenfeld J. et *al.,* précité) sur un appareil de référence Procise 492 vendu par la société Perkin-Elmer.

Les séquences sont ensuite comparées à celles contenues dans les bases de données suivantes : GenBank CDS translation, PDB (*Protein Data Bank*), SwissProt, PIR (Protein Information Resource), PRF (*Protein Research Foundation*), en utilisant le programme BLAST 2.2 (*Basic Local Alignment Search Tool*) de NCBI *(National Center for Biotechnology Information).*

Cette analyse a permis de montrer que la fraction protéique de la partie active est constituée majoritairement de peptides du milieu laitier (lactoferrine, Beta-lactoglobuline, Sérum albumine...), d'une partie qui présente une bonne homologie (60% minimum) avec une séquence de protéine de *Bifidobacterium longum* (RELGIGTPSFLHNGGQWYIYA (SEQ ID n°1), et d'une partie dont il n'a pas été possible de déterminer d'homologie significative avec des séquences connues et possédant les séquences suivantes :
- RVLYNPGQYXYVR (SEQ ID n°2)
- EQATANGQVSSGQQSTGGSAAP (SEQ ID n°3)

### EXEMPLE 3 : ETUDE DE L'ACTIVITE IMMUNOMODULATRICE DE LA FRACTION EXCLUE DU RETENTAT

### 1) Effet de la fraction exclue sur la flore intestinale des souris

L'effet de la fraction exclue (partie active), obtenue selon le procédé décrit ci-dessus à l'exemple 1, sur l'évolution de la flore intestinale des souris a été étudié.

Le test a été réalisé sur des souris mâles C3H qui sont des souris de deuxième génération d'une lignée d'animaux axéniques, auxquelles est implantée une flore intestinale humaine adulte (provenant du CDTA, Centre de Distribution, Typage & Archivage animal, CNRS, Orléans, France).

Les souris sont maintenues dans des isolateurs pour éviter toute modification de leur nouvelle flore intestinale. A leur réception, les souris sont âgées de 8-10 semaines. Un temps d'adaptation d'au moins 2 semaines est laissé aux souris après réception pour supprimer tout stress dû au transport et pour qu'elles s'acclimatent à leur nouvel environnement.

Pendant toute cette durée d'adaptation et jusqu'au début de l'expérience, ces souris disposent d'eau stérile comme boisson.

Pendant toute la durée de l'expérience, la nourriture utilisée comme base alimentaire est un régime standard de granulés RO3 vendus par la société UAR stérilisés par irradiation, contenant 25 % de protéines, 49,8 % de glucides, 5 % de lipides et 4 % de cellulose.

Pendant les 21 jours d'essai, l'eau des biberons est remplacée par les différents produits à tester c'est-à-dire la fraction filtrée, et la fraction exclue, à raison de 6 ml par jour et par souris. Un changement quotidien des biberons est réalisé afin d'éviter toute modification de la composition des produits à tester par prolifération bactérienne. Les selles sont récoltées pour l'analyse avant le traitement (T0), puis au 7^{ème}, au 15^{ème} et au 21^{ème} jour durant l'administration du produit (T7, T15 et T21).

Chaque produit est testé sur un lot d'au moins 6 souris et on suit l'évolution des bactéries *Bifidobacterium* et *Bacteroïdes fragilis* dans la flore intestinale des souris.

Les prélèvements de selles sont faits dans des tubes à hémolyse stériles et sont pesés aseptiquement puis dilués en solution préréduite de Ringer diluée au quart et supplémentée en chlorhydrate de cystéine (0,3 g/l), de façon à obtenir des dilutions au dixième allant de 10⁻¹ à 10⁻⁴. Le contenu est enfin déposé à raison de 100 µl par boîte, sur différents milieux de culture contenus dans des boîtes de Pétri :
- Milieu de Beerens (Be) (composé de 35 g/l de base gélose Columbia, 5 g/l de glucose, 0,3 g/l de chlorhydrate de cystéine, 0,5 % d'acide propionique et ajusté à pH 5) pour la recherche et le dénombrement de *Bifidobacterium,* après étalement de dilutions allant de 10⁻¹ à 10⁻⁴;
- Milieu Bacteroïdes Bile Esculine (BBE) (composé de 37 g/l de tryptone, 32 g/l de *Bile Esculin Agar* (DIFCO), 0,5 g/l d'esculine, 0,5 g/l de citrate de fer ammoniacal, 0,2 % d'une solution d'hémine à 5 mg/ml, 0,25 % d'une solution de gentamicine à 40 mg/ml et 10 g/l d'agar ; ajusté à pH .7 et autoclavé 15 minutes à 120 °C) pour la recherche et le dénombrement de *Bacteroïdes fragilis,* après étalement de dilutions allant de 10⁻¹ à 10⁻³.

L'étalement est réalisé à l'aide de billes de verre stériles sur les milieux indiqués et la lecture des milieux est réalisée après 5-7 jours d'incubation en anaérobiose à 37°C.

L'identification des bactéries est réalisée d'une part après description des colonies obtenues sur chacun des milieux et d'autre part à l'aide d'une coloration de Gram.

Les résultats concernant l'évolution de *Bifidobacterium* et de *Bacteroïdes fragilis* dans les selles des souris sont reportés respectivement dans les Tableaux II et III suivants :

**TABLEAU II**

| *Bifidobacterium* | | | | |
|---|---|---|---|---|
| | **T0** | **T7** | **T15** | **T21** |
| **Fraction filtrée** | 3,8 ±0,5 (9) | 4,3 ± 0,55 (11) | 4,75 ±1,15 (9) * | nd |
| | n=18 | n=18 | n=18 | - |
| **Fraction exclue** | 4,02 ± 0,74 (8) | 4,54 ± 1,0 (11) | 4,56 ± 0,36 (12)* | 4,76 ± 0,27 (6)* |
| | n = 18 | n= 18 | n= 12 | n=6 |

| | | | | |
|---|---|---|---|---|
| nd : non déterminé, *: p<0,025 | | | | |

**TABLEAU III**

| *Bacteroïdes fragilis* | | | | |
|---|---|---|---|---|
| | **T0** | **T7** | **T15** | **T21** |
| **Fraction filtrée** | 4,4 ± 0,7 (15) | 4,4 ± 0,5 (12) | 4,3 ± 0,7 (12) | 4,1 ± 0,9 (7) |
| | n=18 | n=18 | n=18 | n=12 |
| **Fraction exclue** | 4,28 ± 0,35 (18) | 3,99 ± 0,37 (8) | 3,74 ± 0,28 (9) | 3,2 ± 0,16 (4)** |
| | n= 18 | n= 18 | n= 12 | n=6 |

| | | | | |
|---|---|---|---|---|
| nd : non déterminé, * : p<0,025 ; ** : p<0,05 | | | | |

Dans ces tableaux, les résultats obtenus sont exprimés en moyenne et écart-type du log du nombre d'UFC/g de selles, et n est le nombre de souris utilisées pour chaque produit et jours testés (comparaison de rang par test de Wilcoxon pour échantillons appariés), les astérisques indiquent les résultats significatifs par rapport au temps T0. Dans ces tableaux, le chiffre mentionné entre parenthèse correspond au nombre de souris dans lesquelles est présente la bactérie en question au dessus du seuil de détection. Les résultats correspondants au milieu non ensemencé sont similaires à la valeur obtenue à T0 et restent constants au cours du temps pendant les 21 jours de l'expérience (résultats non représentés dans les tableaux).

Ces résultats montrent que l'administration de l'une quelconque des deux fractions (exclue ou filtrée) conduit effectivement à une augmentation des *Bifidobacterium* mais que seule la fraction exclue est efficace pour provoquer une diminution de la population en *Bacteroïdes fragilis* au sein de la flore intestinale des souris.

### 2) Effet de la fraction exclue sur la régulation de la translocation intestinale des micro-organismes

La mesure de la translocation intestinale des micro-organismes chez la souris est effectuée sur les souris mâles de lignée C3H à flore humaine adulte (élevage au CDTA-CNRS, Orléans, France). Pendant toute la durée d'adaptation et jusqu'au début de l'expérience, ces souris disposent d'eau stérile comme boisson.

Pendant toute la durée de l'expérience, ces souris seront nourries avec des granulés RO3 stérilisés par irradiation.

A l'âge de 10-12 semaines, début de l'expérience, on constitue trois groupes, un groupe de 18 souris qui recevra pendant toute la durée de l'expérience la fraction exclue des métabolites de *Bifidobacterium breve* CNCM 1-2219 à la place de l'eau des biberons, à raison de 8-10 ml par jour et par souris pendant que deux autres groupes de 12 et 23 souris recevront respectivement la fraction filtrée ou continueront de recevoir de l'eau stérile (groupe contrôle).

Au 21^{ème} jour, on effectue un prélèvement de selles comme décrit précédemment et les souris sont sacrifiées pour réaliser une étude bactériologique.

Deux types de données sont obtenus à partir de l'analyse des organes des souris sacrifiées:
- la translocation par organe cible, c'est-à-dire l'évaluation du nombre de souris ayant l'organe cible contaminé et du pourcentage de la population présentant une contamination de l'organe cible.
- la dissémination bactérienne, c'est-à-dire l'intensité de la dissémination bactérienne représentée par le nombre d'organes positifs ainsi que le nombre de souris et le pourcentage de la population présentant une dissémination d'une intensité donnée.

Afin de prélever les organes à analyser, l'animal est extrait de l'isolateur, transféré dans des conditions aseptiques sous une hotte à flux laminaire, et sacrifié par inhalation de chloroforme.

La peau est décontaminée avec de l'alcool à 70°, puis sur chaque souris les organes sont prélevés de façon aseptique dans l'ordre suivant : sang du coeur, poumon, foie, rate et rein. Afin d'en évaluer le poids, une fraction de chaque organe est mise en suspension dans 9 ml d'une solution de Ringer (diluée au quart, supplémentée en chlorhydrate de cystéine (0,3 g/l) et régénérée en eau bouillante pendant 15 minutes). Les organes sont ensuite broyés à l'aide d'une pipette Pasteur stérile à usage unique « Pastette® » vendue par la société VWR ou « Liquipette® » vendue par la société Gosselin. Ensuite, 100 µl de la suspension mère et de la dilution décimale suivante sont étalés à l'aide de billes de verre stériles sur gélose Columbia (vendue par la société Beckton-Dickinson), supplémentée en glucose (5 g/l), en chlorhydrate de cystéine (0,3 g/l) et en sang de cheval (5 % v/v, vendu par la société Eurobio). Après une incubation en anaérobiose pendant 7 jours à 37°C, on effectue le dénombrement de chaque type de colonies puis on détermine la morphologie des bactéries après une coloration Gram.

Les résultats obtenus, relatifs à l'analyse de la translocation par organe cible, sont regroupés dans le Tableau IV suivant :

**TABLEAU IV**

| **Organe cible** | **Groupe contrôle** | **Fraction filtrée** | **Fraction exclue** | **p^{c}** |
|---|---|---|---|---|
| **Rein** | 12^{a}(52,2)^{b} | 8 (66,7) | 7 (38,9) | NS |
| **Rate** | 13 (56,5) | 9 (75,0) | 3 (16,7)* | P<0,01 |
| **Foie** | 10 (43,5) | 8 (66,7) | 5 (27,8) | NS |
| **Poumon** | 11 (47,8) | 7 (58,3) | 2 (11,1)* | P<0,02 |

| | | | | |
|---|---|---|---|---|
| ^{a} = nombre de souris ayant l'organe cible contaminé ^{b} = pourcentage de la population présentant une contamination de l'organe cible ^{c} = comparaison des groupes ayant consommé un des produits par rapport au groupe contrôle à l'aide du test exact de Fisher. | | | | |

Le groupe présentant une différence significative est marqué d'une astérisque *.

Les résultats ainsi obtenus montrent que la rate et le poumon sont moins fréquemment contaminés dans la population pour laquelle l'eau de boisson a été substituée par la fraction exclue que dans la population contrôle de souris ayant reçu de l'eau comme boisson.

Par ailleurs, ces résultats font apparaître que la fraction filtrée est sans effet sur la régulation de la translocation bactérienne.

Les résultats obtenus concernant la dissémination bactérienne sont regroupés dans le Tableau V suivant :

**TABLEAU V**

| **Intensité de dissémination** | **Groupe contrôle** | **Fraction filtrée** | **Fraction exclue** | **P^{d}** |
|---|---|---|---|---|
| **Faible (0-1)^{a}** | 8^{b} (35)^{c} | 3 (25) | 12 (66,7)* | P<0,043 |
| **Moyenne (2)** | 4 (17) | 1 (8,3) | 5 (27,8) | NS |
| **Forte (3-4)** | 11 (48) | 8 (66,7) | 1 (5,5)* | P<0,004 |

| | | | | |
|---|---|---|---|---|
| ^{a} = intensité de la dissémination bactérienne représentée (entre parenthèses) par le nombre d'organes positifs, ^{b} = nombre de souris présentant une dissémination d'une intensité donnée, ^{c} = pourcentage de la population présentant une dissémination d'une intensité donnée, ^{d} = comparaison des groupes ayant consommé un des produits par rapport au groupe contrôle à l'aide du test exact de Fisher. Le groupe présentant une différence significative est marqué d'une astérisque *. | | | | |

Le nombre de souris présentant moins d'un organe contaminé (intensité de dissémination faible) est plus élevé dans la population pour laquelle l'eau de boisson a été substituée par la fraction exclue, que dans la population contrôle de souris ayant reçu de l'eau comme boisson.

En outre, le nombre de souris présentant au moins trois organes contaminés (intensité de dissémination forte) est plus faible dans la population pour laquelle l'eau de boisson a été substituée par la fraction exclue, que dans la population contrôle de souris ayant reçu de l'eau comme boisson.

On constate donc que la dissémination des bactéries est moins intense chez les souris ayant reçu de l'eau de boisson complémentée par la fraction exclue. Dans ce cas, la régulation de la translocation bactérienne est plus efficace.

### 3) Effet de la fraction exclue sur l'expression des galectines 1 et 3

La mesure de l'expression des galectines-1 et -3 est effectuée sur les souris à flore humaine adulte décrites précédemment.

A l'âge de 12-14 semaines, début de l'expérience, on constitue deux groupes de souris, un groupe de souris qui recevra pendant toute la durée de l'expérience la fraction exclue contenant les métabolites de *Bifidobacterium breve* CNCM I-2219 à la place de l'eau des biberons, à raison de 10 ml par jour et par souris pendant qu'un autre groupe de souris continuera de recevoir de l'eau (groupe contrôle).

Aux 7^{ème}, 15^{ème} et 21^{ème} jours, un lot de souris est sacrifié pour réaliser une étude biologique sur l'expression des galectines-1 et -3, évaluée par le dosage de l'ARNm codant pour ces galectines par RT-PCR quantitative (*Polymerase Chain Reaction*). Les organes prélevés sur les souris du groupe contrôle sacrifiées au 7^{ème}, 15^{ème} et 21^{ème} jour ont été rassemblés par organe afin de constituer un pool servant de contrôle pendant toute la durée de l'expérience.

La modification de l'expression des galectines-1 et -3 dans les organes testés constitue un indicateur de l'effet des métabolites produits par la souche de *Bifidobacterium breve* CNCM 1-2219 contenus dans la fraction exclue.

Les galectines-1 et -3 sont dosées dans la rate et les poumons de souris ayant été sacrifiées comme décrit précédemment.

Pour ce faire, une fraction de poumon (un lobe) et la moitié de la rate sont prélevées stérilement et dans cet ordre sur chaque souris. Les fragments sont ensuite déposés dans des boîtes de Pétri préalablement traitées pendant 24 heures avec une solution stérile d'eau et de DEPC (DiEthyl PyroCarbonate, vendu par la société Sigma) à 1/1000. Les organes sont alors perfusés une première fois avec du tampon PBS à l'aide d'une seringue et d'une aiguille stérile afin d'en éliminer le sang. Les organes sont ensuite transférés dans une autre boîte de Pétri contenant 200 microlitres de PBS additionné de 50 unités d'inhibiteur de RNase (vendu par la Société Applied Biosystems) et sont perfusés à nouveau avec cette solution. Les organes sont enfin découpés en tranches inférieures à 5 mm qui sont déposées dans un tube Biopur® (Eppendorf) contenant 0,5 ml d'une solution de stabilisation de l'ARN, vendue sous la référence RNALater® par la société Qiagen. Les organes sont ensuite stockés au congélateur à -20°C jusqu'à l'analyse.

### a) Extraction des ARN totaux des organes prélevés

L'ARN total est extrait des tissus après lyse cellulaire selon le protocole d'utilisation du kit d'extraction RNeasy protect® vendu sous la référence 74126 par la société Qiagen.

Pour ce faire, la solution de stabilisation est retirée et une bille de tungstène traitée au DEPC ainsi que 600 microlitres d'une solution de lyse (tampon RLT contenu dans le kit) sont ajoutés à chaque organe. Ils sont ensuite broyés à l'aide d'un broyeur RETSCH MM2000, puis centrifugés à 13000 g pendant 3 minutes à 4°C. Le surnageant est alors placé dans un tube de 1,5 ml contenant 600 microlitres d'éthanol à 70 % puis homogénéisé. Une partie de cette solution (700 microlitres) est ensuite déposée sur une colonne de filtration qui permet d'accrocher les ARN, pour être centrifugée à 8000 g pendant 1 minute à 4°C. Après avoir vidé le tube collecteur, 700 microlitres d'un tampon de lavage (tampon RW1 contenu dans le kit) sont ajoutés sur la colonne qui sera centrifugée à 8000 g pendant 1 minute à 4°C. Le tube collecteur est à nouveau vidé et 500 microlitres d'un second tampon de lavage contenant de l'éthanol à 70 % (tampon RPE contenu dans le kit) est ajouté sur la colonne pour être centrifugée à 8000 g pendant 1 minute à 4°C. Cette dernière étape est renouvelée mais en effectuant une centrifugation à 13 000 g pendant 2 minutes à 4°C. Enfin, 30 microlitres d'un tampon d'élution permettant de décrocher les ARN de la colonne (tampon RNase free contenu dans le kit) sont ajoutés sur la colonne. L'éluat est alors centrifugé à 8 000 g pendant 1 minute à 4°C après avoir été incubé à température ambiante pendant 5 minutes. L'étape d'élution est à nouveau effectuée et les échantillons ainsi obtenus sont congelés rapidement à -80°C.

Une quantité équivalente à une unité de DNase, vendue par la société Boehringer, est ajoutée à chaque échantillon qui est ensuite incubé au bain-marie à 37°C pendant 15 minutes.

Afin d'évaluer la quantité d'ARN total des échantillons, ceux-ci sont dilués au 1/8^{ème} dans de l'eau stérile qui est placée dans une microcuve permettant la lecture de l'absorbance à 260 nm sur un spectrophotomètre UV de référence Genquant, vendu par la société Pharmacia. Pour obtenir la concentration en ARN total des échantillons, la valeur de la densité optique (DO) ainsi obtenue est multipliée par le facteur de dilution et par 40 (une unité de DO = 40 microgramme/ml d'ARN).

### b) RT-PCR quantitative utilisant la méthodologie Taq Man®

La RT-PCR quantitative est réalisée à l'aide du kit qPCR^{™} Core Kit vendu par la société Eurogentec dans un thermocycleur de référence Abi Prism 7700 (*Sequence Detection System,* Applied Biosystems) vendu par la société Perkin Elmer. Le mélange réactionnel résumé dans le tableau VI suivant est réalisé :

**TABLEAU VI**

| **Réactifs** | **Volume en µl/tube** | **Concentration finale** |
|---|---|---|
| Tampon PCR (10 x)* | 5 | 1 x |
| MgCl₂ (50 mM) * | 5 | 5 mM |
| dNTP (2,5 mM) * | 6 | 300 µM |
| Amorce sens (10 pM) | 1 | 200 µM |
| Amorce anti-sens (10 pM) | 1 | 200 µM |
| Sonde (10 pM) | 1 | 200 µM |
| Inhibiteur de RNase (40 unités) | 0,5 | 20 unités |
| Polymérase Hot Gold Star (5 unités) | 0,25 | 1,25 unités |
| MuLV (5 unités) | 0,25 | 12,5 unités |
| ARN totaux | 10 | 100 ng/tube |
| Eau pure | qsp 50 µl | |

| | | |
|---|---|---|
| * produit fourni dans le kit qPCR^{™} Core Kit | | |

Le mélange réactionnel est soumis au programme suivant : 10 minutes à 65°C pour éliminer les structures secondaires des ARN, 30 minutes à 42°C pour activer la transcriptase inverse, 10 minutes à 95°C pour activer la polymérase, 15 secondes à 95°C pour dénaturer les brins d'ADN et 1 minute à 61 °C pour l'hybridation et l'élongation à partir des amorces. Quarante cycles sont effectués pour les deux dernières étapes.

L'expression des galectines-1 et -3 est évaluée par rapport à l'expression d'un gène de référence, tel que celui codant pour la β-actine dont l'expression est constante.

Les sondes et les amorces utilisées et choisies grâce au logiciel Primer Express® vendu par la société Perkin Elmer sont les suivantes :
Galectine-1 :
   Sens : 5'-TCA ATC ATG GCC TGT GGT CTG-3' (SEQ ID n° 4)
   Anti-sens : 5'-AAG CTC TTG GCG TCC GAG G-3' (SEQ ID n° 5)
   Sonde : 5'-TCG CCA GCA ACC TGA ATC AAC CTG-3' (SEQ ID n° 6)
Galectine-3 :
   Sens : 5'-AAT GGC AGA CAG CTT TTC G-3' (SEQ ID n° 7)
   Anti-sens : 5'-GAT CAT GGC GTG GTT AGC-3' (SEQ ID n° 8)
   Sonde: 5'-TTC CAC TTT AAC CCC CGC TTC AAT GAG AAC-3' (SEQ ID n° 9)
β-actine :
   Sens : 5'-TGG CGC TTT TGA CTC AGG ATT-3' (SEQ ID n° 10)
   Anti-sens : 5'-GGG ATG TTT GCT CCA ACC AAC-3' (SEQ ID n° 11)
   Sonde : 5'-GCC GTC GCC TTC ACC GTT CCA GTT TTT-3' (SEQ ID n°12)

Afin de valider chaque microplaque soumise aux cycles de PCR, un calibreur est préparé à partir d'organes d'un lot de 6 souris C3H à flore humaine soumises à un régime alimentaire standard (eau stérile et granules RO3) sur lesquelles les poumons et la rate ont été prélevés. Les ARN totaux de chaque organe ont été extraits à l'aide du protocole précédemment décrit pour les organes des lots de souris à tester. Les différents extraits ainsi obtenus sont ensuite rassemblés pour chaque organe afin de constituer un pool servant de calibreur pendant toute la durée de l'analyse.

Lors de l'analyse, les prélèvements à tester sont déposés en duplicate et la gamme étalon réalisée à partir de l'échantillon calibreur est déposée en triple. Une moyenne est alors réalisée à partir de chaque prélèvement et pour l'échantillon calibreur.

Les résultats de l'expression relative de la galectine-1 par rapport à la β-actine sont regroupés dans le Tableau VII suivant :

**TABLEAU VII**

| | **Alimentation** | | | |
|---|---|---|---|---|
| **Organe** | **Contrôle** | **Fraction exclue** | | |
| | | 7 j | 15 j | 21 j |
| **Rate** | n=17^{a} | n=6 | n=6 | n=3 |
| | 2,78^{b} (1,6-3,47)^{c} | 1,15 (0,72-1,36) U = 0 p<0,002 test bilatéral | 4,5 (3,76-6,92) U = 7 p<0,002 test bilatéral | 12,51 (9,68-13,74) U = 0 p<0,002 test bilatéral |
| **Poumon** | n=16 | n=6 | n=4 | n=6 |
| | 1,145 (0,69-1,93) | 0,8 (0,56-1,08) U = 17,5 p<0,05 test bilatéral | 1,72 (1,67-1,88) U = 5 p<0,02 test bilatéral | 1,465 (0,86-1,77) |

Les résultats de l'expression relative de la galectine-3 par rapport à la β-actine sont regroupés dans le Tableau VIII suivant :

**TABLEAU VIII**

| | **Alimentation** | | | |
|---|---|---|---|---|
| **Organe** | **Contrôle** | **Fraction exclue** | | |
| | | 7 j | 15j | 21 j |
| **Poumon** | n=16^{a} | n = 6 | n=3 | n=6 |
| | 1,295^{b} (0,56-2,26)^{c} | 1,04 (0,86-1,16) | 0,76 (0,41-1) U = 8 p<0,05 test unilatéral | 0,91 (0,75-1,31) |

Dans les tableaux VII et VIII :
- ^{a} = nombre de souris par lot ;
- ^{b}= médiane de l'expression relative de galectine ;
- ^{c}= résultats extrêmes d'expression relative de galectine ;
- la valeur désignée par la lettre U est la valeur statistique du test U de Mann-Whitney;
- p indique le seuil de significativité de la méthode.

Ces résultats montrent qu'une augmentation de l'expression de la galectine-1 est observée dans la rate des souris pour lesquelles l'eau de boisson a été substituée par la fraction exclue. L'expression de la galectine-1 (induction de l'apoptose cellulaire, en particulier de lymphocytes T activés) chez les animaux contrôles est deux fois plus élevée par rapport à celle de l'actine, ce qui semble être en relation avec la contamination bactérienne de l'organe (l'expression de la galectine-1 est significativement inférieure en absence de bactéries dans la rate - U = 15, p<0,05 - et elle augmente en fonction du taux de contamination bactérienne lorsqu'il y a des bactéries - corrélation r = 0,77, p<0,05, test de Spearman). L'expression de la galectine-1 se normalise après 7 jours de prise de la fraction exclue, puis augmente ultérieurement d'un facteur 4 à 10 respectivement à 15 et 21 jours de prise (indépendamment de la contamination bactérienne résiduelle de l'organe). Dans les poumons, l'expression relative de la galectine-1 est normale chez les souris contrôle et la diminution faible mais significative après 7 jours de prise de la fraction exclue est suivie d'une augmentation (transitoirement significative à 15 jours de prise) puis d'une normalisation de l'expression de la galectine-1 après 21 jours de prise de la fraction exclue.

Ces résultats montrent également qu'une baisse de l'expression de la galectine-3 est observée dans les poumons des souris pour lesquelles l'eau de boisson a été substituée par la fraction exclue.

L'ensemble de ces résultats démontre que la fraction exclue (produit immunomodulateur obtenu selon le procédé conforme à l'Invention) a un effet immunomodulateur et permet d'augmenter la population de *Bifidobacterium* et de diminuer la population en *Bacteroïdes fragilis.*

## Revendications

1. Produit immunomodulateur **caractérisé par le fait qu'**il est obtenu selon un procédé de préparation comprenant les étapes suivantes :
- l'ensemencement et l'incubation, en conditions aérobies ou anaérobies et à une température comprise entre 30 et 40 °C environ, de *Bifidobacterium* comprenant au moins la souche *Bifidobacterium breve* I-2219 dans un substrat aqueux présentant un pH compris entre 6 et 8 environ, et comprenant au moins les ingrédients suivants :
i) du perméat de lactosérum,
ii) un hydrolysat de protéines de lactosérum,
iii) du lactose
- l'élimination des *Bifidobacterium* du substrat aqueux ;
- l'ultrafiltration du substrat aqueux sur des membranes de filtration ayant un seuil de coupure compris entre 100 et 300 kDa pour obtenir un rétentat concentré ;
- la déshydratation du rétentat concentré ;
- la mise en solution du rétentat déshydraté dans un tampon ;
- la chromatographie d'exclusion sur gel sur colonne présentant un seuil d'exclusion de 600 kDa de la solution du rétentat ;
- la récupération de la fraction exclue à l'issue de la chromatographie qui constitue le produit immunomodulateur.

2. Produit immunomodulateur selon la revendication 1, **caractérisé par le fait que** les *Bifidobacterium* sont ensemencés dans le substrat aqueux à raison de 1.10⁴ à 4.10⁹ unités formant colonies par ml de substrat.

3. Produit immunomodulateur selon la revendication 1 ou 2, **caractérisé par le fait que** la température du substrat est maintenue à une valeur comprise entre 37 et 40°C pendant toute la durée de l'incubation.

4. Produit immunomodulateur selon l'une quelconque des revendications 1 à 3, **caractérisé par le fait que** le pH du substrat aqueux est maintenu à une valeur comprise entre 6 et 8 pendant toute la période d'incubation.

5. Produit immunomodulateur selon la revendication 4, **caractérisé par le fait que** le pH du substrat aqueux est maintenu à une valeur comprise entre 6,5 et 7,5 pendant toute la période d'incubation.

6. Produit immunomodulateur selon l'une quelconque des revendications précédentes, **caractérisé par le fait que** les ingrédients du substrat aqueux sont présents dans les quantités suivantes :
i) perméat de lactosérum : de 3 à 80 g,
ii) hydrolysat de protéines de lactosérum : de 2 à 80 g,
iii) lactose : de 5 à 50 g
ces quantités étant données par litre dudit substrat aqueux.

7. Produit immunomodulateur selon la revendication 6, **caractérisé par le fait que** les ingrédients du substrat aqueux sont présents dans les quantités suivantes :
i) perméat de lactosérum : de 40 à 60 g,
ii) hydrolysat de protéines de lactosérum : de 5 à 15g,
iii) lactose : de 10 à 30 g
ces quantités étant données par litre dudit substrat aqueux.

8. Produit immunomodulateur selon l'une quelconque des revendications précédentes, **caractérisé par le fait que** le substrat aqueux comprend en outre au moins un ingrédient additionnel choisi parmi les sels tampon, les extraits de levure et le chlorhydrate de cystéine.

9. Produit immunomodulateur selon la revendication 8, **caractérisé par le fait que** le substrat aqueux comprend un sel tampon choisi parmi le dihydrogénophosphate de sodium et le dihydrogénophosphate de potassium qui représente de 0,5 à 5 g par litre de substrat aqueux.

10. Produit immunomodulateur selon la revendication 8, **caractérisé par** le fait l'extrait de levure représente de 0,5 à 5 g par litre de substrat aqueux.

11. Produit immunomodulateur selon la revendication 8, **caractérisé par le fait que** le chlorhydrate de cystéine représente de 100 à 500 mg par litre de substrat aqueux.

12. Produit immunomodulateur selon l'une quelconque des revendications précédentes, **caractérisé par le fait que** l'élimination des *Bifidobacterium* du milieu de culture est réalisée par microfiltration ou par centrifugation du substrat aqueux.

13. Produit immunomodulateur selon la revendication 12, **caractérisé par le fait que** l'élimination des *Bifidobacterium* du milieu de culture est réalisée par centrifugation du substrat aqueux.

14. Produit immunomodulateur selon l'une quelconque des revendications précédentes, **caractérisé par le fait que** le procédé comporte en outre, après l'étape d'élimination des *Bifidobacterium,* une étape supplémentaire de destruction des activités enzymatiques résiduelles contenues dans le substrat aqueux après incubation.

15. Produit immunomodulateur selon l'une quelconque des revendications précédentes, **caractérisé par le fait que** la chromatographie d'exclusion est réalisée sur un gel de dextrane et d'agarose réticulé.

16. Produit immunomodulateur selon l'une quelconque des revendications précédentes, **caractérisé par le fait que** la fraction exclue est essentiellement constituée d'un complexe de polysaccharides et de protéines dans lequel la fraction glucidique représente de 5 à 30 % en poids, la fraction protéique représentant de 70 à 95 % en poids par rapport au poids total dudit complexe.

17. Produit immunomodulateur selon la revendication 16, **caractérisé par le fait que** la fraction glucidique de la fraction exclue présente la composition en monosaccharides suivante (exprimée en rapports molaires par rapport au rhamnose) : galactose : 5,5 à 8 ; mannose : 0,8 à 1,3 ; glucose : 2,5 à 5 ; N-acétyl galactosamine : 0,3 à 1 ; N-acétyl glucosamine : 0,07 à 0,3 ; acide neuraminique 0 à 0,15 et rhamnose : 1.

18. Produit immunomodulateur selon la revendication 16, **caractérisé par le fait que** la fraction protéique comprend au moins un peptide répondant à au moins l'une des séquences suivantes :
- RELGIGTPSFLHNGGQWYIYA (SEQ ID n°1)
- RVLYNPGQYXYVR (SEQ ID n°2)
- EQATANGQVSSGQQSTGGSAAP (SEQ ID n°3)

19. Produit immunomodulateur selon l'une quelconque des revendications précédentes, à titre de médicament.

20. Produit immunomodulateur selon l'une quelconque des revendications précédentes, à titre de médicament immunomodulateur.

21. Composition pharmaceutique **caractérisée par le fait qu'**elle renferme, à titre de principe actif, au moins un produit immunomodulateur obtenu selon le procédé tel que défini à l'une quelconque des revendications 1 à 18, et au moins un support pharmaceutiquement acceptable.

## Claims

1. An immunomodulatory product, **characterized in that** it is obtained according to a method of preparation comprising the following steps:
- inoculation and incubation, under aerobic or anaerobic conditions and at a temperature of between approximately 30 and 40°C, of *Bifidobacterium* comprising at least the strain *Bifidobacterium breve* 1-2219 in an aqueous substrate having a pH of between approximately 6 and 8 and comprising at least the following ingredients:
i) lactoserum permeate,
ii) a lactoserum protein hydrolyzate,
iii) lactose,
- removal of the *Bifidobacterium* from the aqueous substrate;
- ultrafiltration of the aqueous substrate through filtration membranes having a cut-off threshold of between 100 and 300 kDa, so as to obtain a concentrated retentate;
- dehydration of the concentrated retentate,
- dissolution of the dehydrated retentate in a buffer;
- gel exclusion chromatography of the retentate solution, on a column having an exclusion threshold of 600 kDa;
- recovery of the excluded fraction at the end of the chromatography, which fraction constitutes the immunomodulatory product.

2. The immunomodulatory product as claimed in claim 1, **characterized in that** the *Bifidobacterium* bacteria are inoculated into the aqueous substrate in a proportion of 1 x 10⁴ to 4 x 10⁹ colony forming units per ml of substrate.

3. The immunomodulatory product as claimed in claim 1 or 2, **characterized in that** the temperature of the substrate is maintained at a value of between 37 and 40°C throughout the incubation period.

4. The immunomodulatory product as claimed in any one of claims 1 to 3, **characterized in that** the pH of the aqueous substrate is maintained at a value of between 6 and 8 throughout the incubation period.

5. The immunomodulatory product as claimed in claim 4, **characterized in that** the pH of the aqueous substrate is maintained at a value of between 6.5 and 7.5 throughout the incubation period.

6. The immunomodulatory product as claimed in any one of the preceding claims, **characterized in that** the ingredients of the aqueous substrate are present in the following amounts:
i) lactoserum permeate: from 3 to 80 g,
ii) lactoserum protein hydrolyzate: from 2 to 80 g,
iii) lactose: from 5 to 50 g, these amounts being given per liter of said aqueous substrate.

7. The immunomodulatory product as claimed in claim 6, **characterized in that** the ingredients of the aqueous substrate are present in the following amounts:
i) lactoserum permeate: from 40 to 60 g,
ii) lactoserum protein hydrolyzate: from 5 to 15 g,
iii) lactose: from 10 to 30 g, these amounts being given per liter of said aqueous substrate.

8. The immunomodulatory product as claimed in any one of the preceding claims, **characterized in that** the aqueous substrate also comprises at least one additional ingredient chosen from buffer salts, yeast extracts and cysteine hydrochloride.

9. The immunomodulatory product as claimed in claim 8, **characterized in that** the aqueous substrate comprises a buffer salt chosen from sodium dihydrogen phosphate and potassium dihydrogen phosphate, which represents from 0.5 to 5 g per liter of aqueous substrate.

10. The immunomodulatory product as claimed in claim 8, **characterized in that** the yeast extract represents from 0.5 to 5 g per liter of aqueous substrate.

11. The immunomodulatory product as claimed in claim 8, **characterized in that** the cysteine hydrochloride represents from 100 to 500 mg per liter of aqueous substrate.

12. The immunomodulatory product as claimed in any one of the preceding claims, **characterized in that** the removal of the *Bifidobacterium* from the culture medium is carried out by microfiltration or by centrifugation of the aqueous substrate.

13. The immunomodulatory product as claimed in claim 12, **characterized in that** the removal of the *Bifidobacterium* from the culture medium is carried out by centrifugation of the aqueous substrate.

14. The immunomodulatory product as claimed in any one of the preceding claims, **characterized in that** the method also comprises, after the *Bifidobacterium* removal step, an additional step consisting of destruction of the residual enzymatic activities contained in the aqueous substrate after incubation.

15. The immunomodulatory product as claimed in any one of the preceding claims, **characterized in that** the exclusion chromatography is carried out on a crosslinked agarose and dextran gel.

16. The immunomodulatory product as claimed in any one of the preceding claims, **characterized in that** the excluded fraction essentially consists of a complex of polysaccharides and of proteins in which the carbohydrate fraction represents from 5 to 30% by weight, the protein fraction representing from 70 to 95% by weight relative to the total weight of said complex.

17. The immunomodulatory product as claimed in claim 16, **characterized in that** the carbohydrate fraction of the excluded fraction has the following monosaccharide composition (expressed as molar ratios with respect to rhamnose): galactose: 5.5 to 8; mannose: 0.8 to 1.3; glucose: 2.5 to 5; N-acetylgalactosamine: 0.3 to 1; N-acetylglucosamine: 0.07 to 0.3; neuraminic acid: 0 to 0.15, and rhamnose: 1.

18. The immunomodulatory product as claimed in claim 16, **characterized in that** the protein fraction comprises at least one peptide corresponding to at least one of the following sequences:
- RELGIGTPSFLHNGGQWYIYA (SEQ ID No. 1)
- RVLYNPGQYXYVR (SEQ ID No. 2)
- EQATANGQVSSGQQSTGGSAAP (SEQ ID No. 3).

19. The immunomodulatory product as claimed in any one of the preceding claims, as a medicament.

20. The immunomodulatory product as claimed in any one of the preceding claims, as an immunomodulatory medicament.

21. A pharmaceutical composition, **characterized in that** it contains, as active principle, at least one immunomodulatory product obtained according to the method as defined in any one of claims 1 to 18, and at least one pharmaceutically acceptable carrier.

22. The pharmaceutical composition as claimed in claim 21, **characterized in that** it is intended for oral administration and **in that** it is in the form of a liquid or of a solid.

## Patentansprüche

1. Immunregulatorisches Produkt, **dadurch gekennzeichnet, dass** es nach einem Herstellungsverfahren erhalten wird, das die folgenden Schritte umfasst:
- Beimpfen mit und Inkubieren von *Bifidobacterium,* das wenigstens den Stamm *Bifidobacterium breve* I-2219 umfasst, unter aeroben oder anaeroben Bedingungen bei einer Temperatur zwischen ungefähr 30 und 40°C, in einem wässrigen Substrat mit einem pH zwischen 6 und 8 und das wenigstens die folgenden Bestandteile enthält:
i) ein Molkepermeat,
ii) ein Hydrolysat aus Molkeproteinen,
iii) Lactose
- Entfernen des Bifidobakteriums aus dem wässrigen Substrat;
- Ultrafiltrieren des wässrigen Mediums mit Filtermembranen, die eine Ausschlussgrenze zwischen 100 und 300 kDa umfassen, zum Erhalt eines konzentrierten Retentats;
- Dehydrieren des konzentrierten Retentats;
- Lösen des dehydrierten Retentats in einem Puffer;
- Gel-Permeations-Chromatographie der Retentatlösung in einer Säule, die eine Ausschlussgrenze von 600 kDa aufweist;
- Gewinnen der Ausschlussfraktion am Ende der Chromatographie, die das immunregulatorische Produkt darstellt.

2. Immunregulatorisches Produkt nach Anspruch 1, **dadurch gekennzeichnet, dass** das Bifidobakterium mit 1x10⁴ bis 4x10⁹ kolonienbildenden Einheiten pro ml Substrat in das wässrige Substrat eingeimpft wird.

3. Immunregulatorisches Produkt nach Anspruch 1 oder 2, **dadurch gekennzeichnet, dass** die Temperatur des Substrats für die Gesamtdauer der Inkubation auf einem Wert zwischen 37 und 40°C gehalten wird.

4. Immunregulatorisches Produkt nach einem der vorherigen Ansprüche, **dadurch gekennzeichnet, dass** der pH des wässrigen Substrats für die Gesamtdauer der Inkubation auf einem Wert zwischen 6 und 8 gehalten wird.

5. Immunregulatorisches Produkt nach Anspruch 4, **dadurch gekennzeichnet, dass** der pH des wässrigen Substrats für die Gesamtdauer der Inkubation auf einem Wert zwischen 6,5 und 7,5 gehalten wird.

6. Immunregulatorisches Produkt nach einem der vorherigen Ansprüche, **dadurch gekennzeichnet, dass** die Bestandteile des wässrigen Substrats in den folgenden Mengen vorliegen:
i) Molkepermeat: 3 bis 80 g,
ii) Hydrolysat der Molkeproteine: 2 bis 80 g,
iii) Lactose: 5 bis 50 g
wobei diese Mengen pro Liter Substrat angegeben werden.

7. Immunregulatorisches Produkt nach Anspruch 6, **dadurch gekennzeichnet, dass** die Bestandteile des wässrigen Substrats in den folgenden Mengen vorliegen:
i) Molkepermeat: 40 bis 60 g,
ii) Hydrolysat der Molkeproteine: 5 bis 15 g,
iii) Lactose: 10 bis 30 g
wobei diese Mengen pro Liter Substrat angegeben werden.

8. Immunregulatorisches Produkt nach einem der vorherigen Ansprüche, **dadurch gekennzeichnet, dass** das wässrige Substrat wenigstens einen zusätzlichen Bestandteil, ausgewählt aus Puffersalzen, Hefeextrakten und Cysteinchlorhydrat umfasst.

9. Immunregulatorisches Produkt nach Anspruch 8, **dadurch gekennzeichnet, dass** das wässrige Substrat ein Puffersalz, ausgewählt aus Natriumdihydrogenphosphat und Kaliumdihydrogenphosphat, umfasst, das in einer Menge von 0,5 bis 5 g pro Liter des wässrigen Substrats vorliegt.

10. Immunregulatorisches Produkt nach nach Anspruch 8, **dadurch gekennzeichnet, dass** das Hefeextrakt in einer Menge von 0,5 bis 5 g pro Liter des wässrigen Substrats vorliegt.

11. Immunregulatorisches Produkt nach nach Anspruch 8, **dadurch gekennzeichnet, dass** das Cysteinchlorhydrat in einer Menge von 100 bis 500 mg pro Liter des wässrigen Substrats vorliegt.

12. Immunregulatorisches Produkt nach einem der vorherigen Ansprüche, **dadurch gekennzeichnet, dass** das Entfernen des Bifidobakteriums aus dem Kulturmedium durch Mikrofiltration oder durch Zentrifugation des wässrigen Mediums erfolgt.

13. Immunregulatorisches Produkt nach Anspruch 12, **dadurch gekennzeichnet, dass** das Entfernen des Bifidobakteriums aus dem Kulturmedium durch Zentrifugation des wässrigen Mediums erfolgt.

14. Immunregulatorisches Produkt nach einem der vorherigen Ansprüche, **dadurch gekennzeichnet, dass** das Verfahren unter anderem, nach dem Schritt des Entfernens des Bifidobakteriums, einen zusätzlichen Schritt des Zerstörens der im wässrigen Substrat nach dem Inkubieren vorhandenen enzymatischen Restaktivität beinhaltet.

15. Immunregulatorisches Produkt nach einem der vorherigen Ansprüche, **dadurch gekennzeichnet, dass** die Gel-Permeations-Chromatographie mit einem Dextrangel oder einem vernetzen Agarosegel erfolgt.

16. Immunregulatorisches Produkt nach einem der vorherigen Ansprüche, **dadurch gekennzeichnet, dass** die Ausschlussfraktion im Wesentlichen aus einem Polysaccharid- und einem Proteinkomplex besteht, in dem die kohlenhydrathaltige Fraktion 5 bis 30 Gewichtsprozent und die Proteinfraktion 70 bis 95 Gewichtsprozent im Verhältnis zum Gesamtgewicht des Komplexes darstellt.

17. Immunregulatorisches Produkt nach Anspruch 16, **dadurch gekennzeichnet, dass** die kohlenhydrathaltige Fraktion der Ausschlussfraktion folgende Zusammensetzung an Monosacchariden (ausgedrückt in molaren Verhältnissen im Verhältnis zur Rhamnose) aufweist: Galactose: 5,5 bis 8; Mannose: 0,8 bis 1,3; Glucose: 2,5 bis 5; N-Acetylgalactosamin: 0,3 bis 1; N-Acetylglucosamin: 0,07 bis 0,3; Neuraminsäure: 0 bis 0,15 und Rhamnose: 1.

18. Immunregulatorisches Produkt nach Anspruch 16, **dadurch gekennzeichnet, dass** die Proteinfraktion wenigstens ein Peptid umfasst, das wenigstens einer der folgenden Sequenzen entspricht:
- RELGIGTPSFLHNGGQWYIYA (SEQ ID Nr. 1)
- RVLYNPGQYXYVR (SEQ ID Nr. 2)
- EQATANGQVSSGQQSTGGSAAP (SEQ ID Nr. 3).

19. Immunregulatorisches Produkt als Medikament nach einem der vorherigen Ansprüche.

20. Immunregulatorisches Produkt als immunregulatorisches Medikament nach einem der vorherigen Ansprüche.

21. Pharmazeutische Zusammensetzung, **dadurch gekennzeichnet, dass** sie als aktiven Bestandteil wenigstens ein immunregulatorisches Produkt einschließt, das nach dem Verfahren nach einem der Ansprüche 1 bis 18 erhalten wird, und wenigstens einen pharmazeutisch verträglichen Träger.

22. Pharmazeutische Zusammensetzung nach Anspruch 21, **dadurch gekennzeichnet, dass** sie für die orale Verabreichung bestimmt ist und sich in flüssiger oder fester Form befindet.
